Europäisches Patentamt

European Patent Office (11) Publication number: **0 080 341**

Office européen des brevets **B1**

(19)

(12) **EUROPEAN PATENT SPECIFICATION**

(45) Date of publication of patent specification: **28.10.87**   (51) Int. Cl.⁴: **A 61 K 9/22, A 61 K 9/52**

(21) Application number: **82306170.0**

(22) Date of filing: **19.11.82**

(54) Pharmaceutical multiple-units formulation.

(30) Priority: **20.11.81 DK 5164/81**

(43) Date of publication of application:
**01.06.83 Bulletin 83/22**

(45) Publication of the grant of the patent:
**28.10.87 Bulletin 87/44**

(84) Designated Contracting States:
**AT BE CH DE FR GB IT LI LU NL SE**

(56) References cited:
**EP-A-0 013 263**
**EP-A-0 040 590**
**FR-A-1 503 582**
**FR-A-2 059 978**
**FR-A-2 116 256**
**FR-A-2 197 576**
**FR-A-2 419 722**
**GB-A-2 041 222**
**US-A-3 081 233**
**US-A-4 173 626**
**US-A-4 291 016**

**CHEMICAL ABSTRACTS, vol. 87, no. 26, 26th December 1977, page 284, no. 206452d, Columbus, Ohio, USA P.J. McDONALD et al.: "Studies on absorption of a newly developed enteric-coated erythromycin base"**

(73) Proprietor: **A/S Alfred Benzon**
**Halmtorvet 29**
**DK-1700 Copenhagen V (DK)**

(72) Inventor: **Bechgaard, Helle**
**6, Valbirkvej**
**DK-2900 Hellerup (DK)**
Inventor: **Houmoeller, Peter**
**113, Baunevej**
**DK-2630 Tastrup (DK)**

(74) Representative: **Walls, Alan James**
**Windy Ridge 2, Lywood Close**
**Tadworth Surrey KT20 5SS (GB)**

(56) References cited:
**CHEMICAL ABSTRACT, vol. 85, no. 2, 12th July 1976, page 323, no. 10368v, Columbus, Ohio, USA T. YAMASHITA et al.: "Blood concentration and urinary exretion in humans of OE 7, a gastric hard capsule containing entericoated granules"**

**MANUFACTURING CHEMIST & AEROSOL NEWS, vol. 41, no. 5, May 1970, pages 53-57, London, GB. B.E. JONES: "Production of enteric coated capsules"**

Courier Press, Leamington Spa, England.

(56) References cited:

CHEMICAL ABSTRACTS, vol. 89, no. 12, 18th
September 1978, page 304, no. 94961b,
Columbus, Ohio, USA Y. NOZAWA et al.: "Drug
dissolution from the microcapsules containing
enteric coating materials"

CHEMICAL ABSTRACTS, vol. 88, no. 14, 3rd
April 1978, page 330, no. 94774x, Columbus,
Ohio, USA R. GURNY et al.: "Realization and
theoretical development of controlled-release
drug forms using methacrylate films. 3.
Preparation and characterization of controlled-
release drug forms".

CHEMICAL ABSTRACTS, vol. 91, no. 20, 12th
November 1979, page 345, no. 162999p,
Columbus, Ohio, USA Y. NOZAWA et al.: "Drug
dissolution from microcapsules containing
enteric coating materials"

CHEMICAL ABSTRACTS, vol. 93, no. 20, 17th
November 1980, page 356, no. 191980x,
Columbus, Ohio, USA B. BERGDAHL et al.:
"Absorption of digoxin from a new
microencapsulated formulation"

CHEMICAL ABSTRACTS, vol. 81, no. 20, 18th
November 1974, page 425, no. 126806h,
Columbus, Ohio, USA

# 0 080 341

**Description**

The present invention relates to an oral pharmaceutical controlled release multiple-units formation with important new features.

Technical background

Many physiological factors influence both the gastrointestinal transit time and the release of a drug from a controlled release dosage form and thus the uptake of the drug into the systemic circulation. Dosage forms should therefore be designed so that such variable factors do not compromise the efficacy and safety of the product.

In humans, a reproducible gastrointestinal transit time of a depot formulation can be achieved only by a controlled release multiple-units formulation.

The term "controlled release multiple-units formulation" (Bechgaard & Hegermann Nielsen, 1978) indicates a pharmaceutical formulation comprising a multiplicity (typically at least 100) of individual coated (or "microencapsulated") units contained in the formulation in such a form that the individual units will be made available from the formulation upon disintegration of the formulation in the stomach of animals, including humans, who have ingested the formulation. Typically, the multiple-units formulation may be a capsule which disintegrates in the stomach to make available a multiplicity of individual coated units contained in the capsule, or a tablet which disintegrates in the stomach to make available a multiplicity of coated units originally combined in the tablet.

Drug release from a controlled release dosage form is generally controlled either by *diffusion* through a coating or by *erosion* of a coating by a process dependent on, e.g., enzymes or pH. The importance of a pH independent diffusion with respect to obtaining a reproducible rate of availability and to minimizing intra- and intersubject variations is known (GB Patent No. 1 468 172 and Bechgaard & Baggesen, 1980). It is also known that controlled drug release *in vivo* can be achieved through an erodable process by enteric coating of a multiple-units formulation (Green, 1966; McDonald *et al.*, 1977; Bogentoft *et al.*, 1978).

Both above-mentioned types of controlled release multiple-units formulation techniques aim at a controlled release of active substance in a predetermined pattern to reduce and delay the peak plasma concentration without affecting the extent of drug availability. Due to a lower peak plasma concentration, the frequency of undesirable side-effects may be reduced, and due to the delay in the time to obtain the peak plasma concentration and the extension of the time at the therapeutically active plasma level, the dosage frequency may be reduced to daily dosage only twice or once, in order to improve patient compliance.

A further advantage of the controlled release multiple-units formulation is that high local concentrations of the active substance in the gastrointestinal system is avoided, due to the units being distributed freely throughout the gastrointestinal tract, independent of gastric emptying. If the mucosa of the stomach is more sensitive to the active substance than the intestinal mucosa, controlled release formulations avoiding release of active substance in the gastric area will be preferred, formulations of this type are controlled release multiple-units formulations in which the coatings are substantially resistant to gastric conditions.

Disclosure of invention

The present invention relates to new developments in controlled release multiple-units formulations where the individual units are coated with an erodable coating; more specifically, the invention relates to such formulations which are directed to improve the solubility of sparingly soluble active substances.

Accordingly, the present invention relates to a pharmaceutical oral controlled release multiple-units formulation which comprises particles comprising an active substance requiring more than 30 parts by volume of water to dissolve 1 part by weight of the active substance at ambient temperature (in the following termed "a sparingly soluble active substance"), in intimate admixture with a dispersion-accelerating substance which is readily soluble in intestinal fluids, which particles are combined into cross-sectionally substantially homogeneous cores together with at least one component which enhances the disintegration of the cores in intestinal fluids, the cores being coated with a coating which is substantially resistant to gastric conditions, but which is erodable under the conditions prevailing in the small intestine.

Pharmaceutical formulations in which a sparingly soluble active substance is combined with a disintegration-enhancing substance and which comprise an erodable coating has been previously been described. For instance, US Patent No. 4,173,626 discloses a formulation comprising a capsule which contains uncoated pellets of indomethacin and one or more pharmaceutical carriers as well as coated pellets of indomethacin and one or more pharmaceutical carriers (and non-medicated pellets). The pellets are prepared by mixing indomethacin with binding agents, a disintegration agent such as corn starch, guar gum, gum tragacanth, agar of sodium starch glycolate, and a filler, and the mixture is granulated and pelleted to form pellets of a size of 0.5—1.5 mm. To produce the final product, some of these pellets are coated with a slowly disintegrating coating, such as polyvinyl acetate, methacrylic and esters, ethyl cellulose, polyvinyl alcohol, etc. However, the US patent does not disclose any intimate admixture of a sparingly soluble active substance and a dispersion-enhancing substance in the form of particles which are mixed with a disintegration-accelerating component (or other pharmaceutical excipients) which, in accordance with the present invention, has a particularly beneficial effect (cf. Example 2 of the present specification).

US Patent No. 2,291,016 discloses a matrix tablet comprising a mixture of an active substance and a surface-active agent (e.g. sodium lauryl sulphate) and optionally binders and fillers. This patent discloses an increased absorption of the active substance in the organism ascribable to the presence of the surface active agent. The tablet is coated with an erodable coating which is dissolved at a pH of 5—8 within 0.25—2 hours. The coating comprises e.g. cellulose acetates phthalate or a methacrylic acid ester. A matrix tablet as disclosed in this US patent passes the stomach as a single unit and disintegrates on erosion of the enteric coating followed by instant release of the active substance in contrast, the present invention relates to a formulation comprising a multiplicity of coated cores (pellets) which on disintegration of the tablet or capsule containing them are freely distributed throughout the gastro-intestinal tract whereby the absorption phase of the active substance is prolonged. Nor does the composition of the tablet disclosed in US Patent No. 4,291,016 lead to any increased absorption of the active substance as it lacks the particles in which the active substance is intimately admixed with the dispersion-accelerating substance which provides the increased absorption effect (cf. Example 2 of the present specification).

European Patent Application, Publication No. 0040590 discloses cores containing an active substance mixed with lactose which cores are coated with an enteric coating. The European patent application does not disclose a composition in which the active substance is intimately admixed with a disintegration-accelerating substance to form particles, which is an important feature of the pellets of the present invention as it contributes to the increased absorption of a sparingly soluble active substance.

The individual units of the multiple-units formulations according to the invention are pellets (coated cores) in which the core is constituted by a combination of active substance and excipients. In the present context, the term "cross-sectionally substantially homogeneous cores" designates cores which, through the cross-section of the core body, contain substantially the same type of composition comprising microparticles containing an active substance. From this definition, it will be understood that the cores which are cross-sectionally substantially homogeneous will normally consist of a mixture of active substance with excipient(s), (and in spite of the term "homogeneous", this mixture will not necessarily be qualitatively or quantitatively homogeneous through the cross-section of the particle but may show, e.g., a concentration gradient of one or more of its constituents). In the following specification and claims, such cores which are cross-sectionally substantially homogeneous will, for the sake of brevity, often simply be designated "cores".

The erodable coatings used in the formulations of the present invention are coatings which are substantially resistant under gastric conditions but are eroded during the passage of the unit through the small intestine. Erodable coatings may be coatings which are eroded by a process dependent on, e.g., enzymes present in the segment of the intestine where the erosion is desired, including enzymes generated by the animal, including the human, to whom the unit is administered and enzymes produced by bacteria, or bacterial fermentation of the erodable coating. As has been explained above, erodable coatings are distinguished from diffusion coatings which are substantially insoluble and non-erodable in gastrointestinal fluids, but are permeable, by diffusion, to gastrointestinal fluids and dissolved active substance. (For the sake of completeness, however, it should be noted that although the quantitatively predominant contribution to the absorption from erodably coated units is the phase following the erosion of the coating it cannot be precluded that a certain amount of active substance will be released through the uneroded coating by diffusion).

An important class of erodable coatings for use in the formulations according to the present invention are the so-called enteric coatings which are coatings that are substantially insoluble under the pH conditions prevailing in the stomach but are soluble at a pH prevailing in the small intestine, typically a pH of above 4.5.

Detailed description of invention
Particles containing an active substance

According to the invention, the sparingly soluble active substances are incorporated in the cores in the form of particles in which they are intimately admixed with a substance which is readily dissolved in intestinal fluids and which, therefore, accelerates the dispersion of the active substance. Such an intimate admixture may be obtained, e.g., by co-comminuting the active substance together with the dispersion-accelerating substance, both substances preferably being in solid form during the comminution. The co-comminution may be performed by subjecting a mixture of particles of the active substance with particles of the dispersion-acclerating substance to grinding, in particular high shear grinding, e.g. in a pinned disc mill or a jet mill or other equipment exerting similar stress. The resulting intimate mixture will be in the form of particles in the range of 1—10 μm, in particular 2—5 μm, in which the active substance and the dispersion-accelerating substance are intimately combined with each other by conglomeration and/or adsorption. The particles in which a sparingly soluble active substance is combined with a dispersion-accelerating substance show an accelerated dissolution of the active substance, which is believed to be due to the fact that the dispersion-accelerating substance incorporated in the particles accelerates the dispersion of the active substance which is thereby more efficiently exposed to the intestinal fluids.

4

The dispersion-accelerating substance which, in accordance with the invention, is incorporated in the particles containing active substance may, in principle, be any pharmaceutically acceptable excipient which is readily soluble in intestinal fluids. Examples of such substances are sucrose, glucose, mannitol, sorbitol or lactose. Especially effective dispersion-accelerating substances are surface-active substances, in particular anionic or non-ionic detergents, for instance sodium salts of fatty alcohol sulphates, preferably sodium laurylsulphate, sulfosuccinates, partial fatty acid esters of sorbitans, e.g. sorbitanmonooleate (SPAN®), partial fatty acid esters of polyhydroxyethylene sorbitans, e.g. polyethylene glycolsorbitan monooleate (Tween® 80), or polyhydroxyethylene fatty alcohol ether, e.g. polyhydroxyethylene (23) lauryl ether (BRIJ® 35).

The amount of dispersion-accelerating substance which is incorporated in the particles containing active substance is normally less than 100%, calculated on the active substance, typically at the most 70%, calculated on the active substance. Thus, for instance, when the readily soluble substance is sucrose or another dispersion-accelerating carbohydrate, it is normally co-comminuted with the active substance in an amount of about 40—60% by weight, calculated on the active substance. When the dispersion-enhancing substance is a surface-active substance such as a detergent, it is preferably co-comminuted with the active substance in an amount of at the most 10% by weight, preferably about 5% by weight, calculated on the active substance.

Cores

According to the invention, the cores are cross-sectionally substantially homogeneous cores. The cores are typically made by granulating the particles containing the active substance in intimate admixture with the dispersion-accelerating substance together with at least one disintegration-enhancing component and optionally other excipients, including bulk agents such as carbohydrates and derivatives thereof such as starch and starch derivatives, including microcrystalline cellulose, binders such as cellulose derivatives, including methylcellulose or hydroxypropylmethylcellulose, polyethylene glycol, polyvinylpyrrolidone, agar, or gelatin, for instance by treatment in a high speed mixer (to directly obtain compact-shaped cores), or by treatment in a planet mixer with subsequent extrusion of the mixture into strings of a predetermined diameter approaching the desired final cross-sectional dimension of the cores and treatment of the strings in a marumerizer or similar equipment to obtain compact-shaped cores. The diameter of the cores is normally adapted so that the diameter of the coated core is 0.4—1.2 mm, in particular 0.5—1.0 mm, especially 0.5—0.8 mm, e.g. 0.5—0.7 mm. A preferred diameter of the coated cores is 0.5—0.6 mm. Examples of disintegration-enhancing components are particulate substances which are insoluble in gastric and intestinal fluids and which tend to counteract excessive compaction of the content of the cores during their preparation, and/or to introduce slidability between the components in the cores, and/or to geometrically introduce irregular stresses in the cores, and/or to interfere with the packing of the content of the cores to provide voids between the particles containing active substance, selected from plate-shaped particles, e.g. talc, or compact-shaped particles of a particle size of about 20—100 μm, in particular about 50—75 μm, e.g. aluminium silicate, zinc oxide, magnesium oxide, titanium dioxide, colloidal silica, or magnesium trisilicate.

It has furthermore been found that the disintegration of the cores is additionally enhanced when particles of a substance which is readily soluble in intestinal fluids are incorporated in the mixture from which the cores are made. Examples of such substances are sucrose, glucose, mannitol, sorbitol, or lactose.

In particular, it is preferred to enhance the disintegration of the cores in intestinal fluids by a combination of the two above-mentioned measures that is, by incorporation of both an insoluble and a soluble disintegration-enhancing component. One example of a combination of this type is the combination of talc and sucrose which is illustrated in the Examples.

In order to obtain maximum disintegration of the cores so that the sparingly soluble active substance contained therein is exposed to the intestinal fluids as effectively and speedily as possible, it is preferred to use only a small amount of binder, if any, in the mixture from which the cores are made.

The amount of disintegration-enhancing components (insoluble and/or soluble) are usually at least 20% by weight, typically at least 40% by weight, calculated on the total mixture of active substances and excipients.

In accordance with a particular aspect of the invention, the predetermined controlled release of the active substance may be altered by changing the density of the cores, and thus, the time of arrival of the cores in the predetermined section of the intestine may be varied at will. By increasing the density of the cores resulting in increased transit time of the coated cores (Bechgaard & Ladefoged, 1978), a more delayed and longer lasting absorption phase is obtained, that is a longer period during which the absorption of the active substance takes place affer the substance has been released by erosion of the coating, thus having become available for absorption.

Examples of excipients which may be used to increase the density of the cores are described in US Patent No. 4 193 985 and include heavy particulate substances such as barium sulphate, titanium oxide, zinc oxides, and iron salts.

Active substance

Examples of sparingly soluble active substances (as defined above) which may be incorporated in the cores are found among almost all therapeutic groups, including diuretics, antiepileptics, sedatives, antiarrhytmics, antirheumatics, β-blockers, vasodilators, analgesics, bronchodilators, hormones, oral antidiabetics, antihypertensives, antiinflammatorics, and antidepressives.

Important sparingly soluble substances belong to a group which requires more than 1000 parts by volume of water to dissolve 1 part by weight of the active substance at ambient temperature, or even more than 10,000 parts by volume of water.

As examples of sparingly soluble active substances which may be formulated according to the invention may be mentioned indomethacin, spironolactone, ibuprofen, furosemide, sulfadiazine, sulfamerazine, progesterone, reserpine, pyrvinium embonate, mofebutazone, hydrochlorothiazide, tetracycline, tolbutamide, acetaminophen, testosterone, valproic acid, estradiol, acetazolamide, erythromycin, iron salts, hydralazine, carbamazepine, quinidine, and cardiac glycosides, e.g., digoxin.

As examples of substances among the above-mentioned sparingly soluble substances which require more than 1000 parts by volume of water to dissolve 1 part by weight of the substance at ambient temperature may be mentioned spironolactone, ibuprofen, furosemide, hydrochlorothiazide, tolbutamide, and testosterone.

By utilizing the principle of the invention, it is possible to obtain an extent of availability of a sparingly soluble active substance which is equal to or better than the extent of availability of plain formulations and to reduce and delay the peak plasma concentration compared to plain formulations. This is achieved by utilizing (i) the fact that the units are freely distributed throughout the gastrointestinal tract independently of gastric emptying, as the units are small enough to pass the pylorus even when the sphincter is closed, and (ii) the fact that there is a significant physiological variation along the length of the gastrointestinal tract, including variation in pH and qualitative and quantitative composition of enzymes and microflora. In the stomach, the pH range is wide, viz. pH 1—6, primarily due to an increase in pH after the intake of food, while the pH in the small intestine ranges from 5 to 8. The variation in the physiological environment along the length of the small intestine may be utilized by adapting the erodable coating to be eroded in a desired segment of the small intestine.

Coating

The erodable coating applied to the cores according to the invention is preferably an enteric coating which is applied from a solution and/or suspension in an organic solvent and/or water. The application of the coating is typically performed in a fluidized bed or by pan coating.

Examples of enteric coating materials which may be used for the purpose of the present invention are coatings selected from the group consisting of acrylic polymers and copolymers, e.g. a polymerisate of methacrylic acid and methacrylic acid methyl ester such as Eudragit® S 12.5. Eudragit® S 12.5 P (which corresponds to Eudragit® S 12.5 but contains 1.25% of dibutyl phthalate), Eudragit® L 30 D or Eudragit® L 12,5, shellac, cellulose acetate esters such as mixed partial esters of cellulose-containing phthalate groups, acetyl groups and free acid groups (cellulose acetate phthalate), polyvinyl acetate esters such as polyvinyl acetate phthalate, hydroxypropylmethyl cellulose esters such as hydroxypropylmethylcellulose phthalate, or alkyleneglycol ether esters of copolymers such as partial ethyleneglycol monomethyl ether ester of ethyl acrylate-maleic anhydride copolymer, propyleneglycol monomethyl ether ester of ethyl acrylate-maleic anhydride copolymer, dipropyleneglycol monomethyl ether ester of ethyl acrylate-maleic anhydride copolymer or diethyleneglycol monomethyl ether ester of methyl acrylate-maleic anhydride copolymer, n-butyl acrylate-maleic anhydride copolymer, isobutyl acrylate-maleic anhydride copolymer or ethyl acrylate-maleic anhydride copolymer.

The coating material may be admixed with various excipients such as plasticizers, inert fillers, e.g. talc, pigments, in a manner known *per se.*

The type and amount of enteric coating applied is adapted so as to obtain resistance to gastric environments and release in the desired segment of the small intestine. Normally, the amount of the coating will be 2—25% by weight, calculated as dry matter on the total weight of the cores, typically 4—12% by weight (for coated cores with a diameter of 0.5—0.6 mm).

In accordance with one aspect of the present invention, the coating is so selected that it will preferably be eroded in the distal part of the small intestine. An sxample of such a coating is an enteric coating which is substantially insoluble at a pH below 7.

In the present context, the term "an enteric coating which is substantially insoluble at a pH below 7" designates an enteric coating which, under the experimental conditions defined under Materials and Methods below, releases less than 15% of the active substance contained in the coated core within one hour at pH 6.5.

Preferably, the coating which is substantially insoluble at a pH below 7 will, at the same time, effectively release a high proportion of the active substance, typically at least 90% of the active substance contained in the core, within one hour at pH 7.5, under the experimental conditions defined under materials and methods below.

Preferred materials for enteric coatings which are substantially insoluble at a pH below 7 are polymerisates of methacrylic acid and methacrylic acid methyl ester or mixtures thereof. A specific example of such a coating material is Eudragit® S.

The use of a coating which is selectively or preferentially eroded in the distal part of the small intestine offers several advantages:

Firstly, due to the longer passage of the units through the small intestine to reach the distal section of the small intestine in which the pH is in the range of 7—8, the time before the peak plasma concentration is reached is prolonged.

It is known that physiological pH variations in the distal segment of the small intestine are small. Furthermore, pH variations caused by food intake are also low in the distal segment. Due to these very stable pH conditions, controlled release formulations of the invention in which the coating is one that is substantially insoluble at pH below 7 will yield a highly reproducible absorption phase, both within and between subjects and are therefore preferred.

As appears from Example 5, the standard deviations of the bioavailability parameters of a formulation according to the invention where the coating is an enteric coating which is substantially insoluble at a pH below 7 were of the same order of magnitude when the formulation was administered with food or after a fast, respectively. The administration of the coated cores concomitantly with food intake did not influence the extent of availability.

The aspect of the invention comprising controlled release multiple-units formulations in which the coating is an erodable coating which erodes selectively in the distal part of the small intestine, in particular an enteric coating which is substantially insoluble at pH below 7, is of general importance and advantage in connection with any type of units.

Brief description of drawing

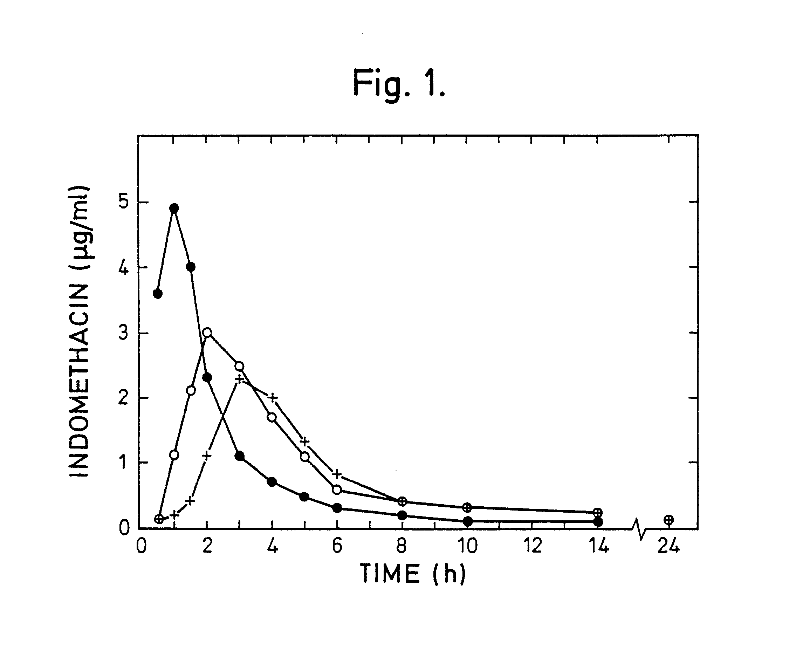

Fig. 1 is a graph showing the mean concentrations of indomethacin in plasma after single oral doses of 75 mg in the form of a reference formulation (indicated by filled-in circles) or in the form of Coating A capsules according to the invention (indicated by circles) or Coating B capsules according to the invention (indicated by crosses). The concentrations are the ones stated in Example 4.

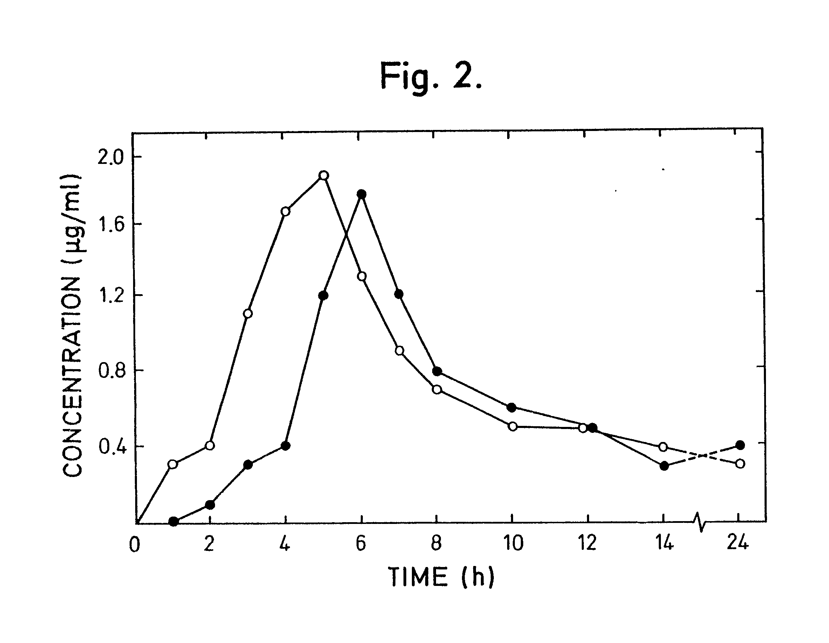

Fig. 2 illustrates the mean plasma concentration of indomethacin after single oral doses of 75 mg as Coating B capsules according to the invention after a twelve-hour fast (indicated by circles) or within 15 min of ingestion of food (indicated by filled-in circles). The concentrations are the ones stated in Example 5.

The invention is illustrated in greater detail in the following experimental section.

Materials and methods

In the examples, the following materials were used:

| | |
|---|---|
| Indomethacin: | (2-[1-(4-Chlorobenzoyl)-5-methoxy-2-methylindol-3-yl]acetic acid); BP 80. |
| Sodium laurylsulphate: | Ph Eur. |
| Microcrystalline cellulose: | BPC 79. |
| Sucrose powder: | Ph Eur. |
| Talc: | Ph Eur. |
| Purified water: | Ph Eur. |
| Eudragit® S 12,5: | An anionic polymerisate of methacrylic acid and methacrylic acid methyl ester having a dry matter content of 12.5%, a density $D^{20}$ of 0.84, a viscosity at 20°C of 100 cP; supplied by Röhm Pharma GmbH, Darmstadt, Germany. |
| Eudragit® L 12,5: | An anionic polymerisate of methacrylic acid and methacrylic acid methyl ester having a dry matter content of 12.5%, a density of $D^{20}$ of 0.84, and a viscosity at 20°C of 75 cP; supplied by Röhm Pharma GmbH, Darmstadt, Germany. |
| Eudragit® L 30 D: | An anionic polymerisate of methacrylic acid and methacrylic acid methyl ester having a dry matter content of 30% as an aqueous dispersion, supplied by Röhm Pharma GmbH, Darmstadt, Germany. |
| Acetyltributylcitrate: | Citroflex® A-4; supplied by Pfizer A/S, Copenhagen, Denmark. |
| Isopropanol: | BP 80. |
| Polyvinylpyrrolidone: | BP 80 Add 81. |
| Purosemide: | (4-Chloro-N-furfuryl-5-sulfamoylantranilic acid) BP 80. |
| Triacetin: | (1,2,3-propanetrioltriacetate) USP XX. |

Determination *in vitro* dissolution of pellets or cores:

*In vitro* dissolution rates were determined according to Baggesen *et al* (1981). The rotation speed was $30 \pm 1$ r.p.m. and the dissolution medium was 250 ml of 0.1 $M$ hydrochloric acid (pH 1.2) or citrate buffer (0.05 $M$, pH 4.5 or 0.02 $M$, pH 6.5) or phosphate buffer (0.05 $M$, pH 7.5), maintained at $37 \pm 0.1$°C. Release of active substance into the dissolution medium was determined by measuring the absorbance spectrophometrically at 320 nm (indomethacin) or 271 nm (furosemide).

Example 1

Preparation of indomethacin-containing cores to be coated with an enteric coating

Cores (containing 24% of talc) were prepared from 2.9 kg indomethacin, 0.2 kg sodium laurylsulphate, 0.5 kg microcrystalline cellulose, 4.0 kg sucrose powder and 2.4 kg talc.

The indomethacin and the sodium laurylsulphate were co-comminuted by passage through a grinder using a 0.5 mm sieve.

The ground mixture was mixed with the microcrystalline cellulose, the sucrose and the talc in a planet mixer.

10 kg of the resulting mixture were moistened with 0.8 kg purified water and were mixed in a planet mixer until the mixture was granular.

The moist mixture was extruded through a 0.5 mm sieve. The first kgs of extrudate passing the sieve were powdery and were reextruded. The resulting extrudate were strings breaking off in lengths of 10—30 cm.

2 kg of the extruded strings were formed into compact-shaped cores in a marumerizer, and the resulting compact-shaped cores were dried in a fluidized bed dryer and then sieved through a separator, the upper sieve being 0.71 mm, and the bottom sieve 0.46 mm.

In a similar manner as described above, cores (containing 10% of talc) were prepared from 2.9 kg indomethacin, 0.2 kg sodium laurylsulphate, 1.0 kg microcrystalline cellulose, 4.0 kg saccharose powder, and 1.0 kg talc.

The release of the indomethacin was measured, at pH 7.5, as described under Materials and Methods, for the cores containing 24% of talc and 10% of talc, respectively. The amount of indomethacin released at pH 7.5 after 10 minutes appears from Table I.

TABLE I

Percentage of indomethacin released at pH=7.5
after 10 min (n=2)

| | |
|---|---|
| Cores with 24% of talc | 98.4% |
| Cores with 10% of talc | 60.0% |

It appears from Table I that the increase of the talc content from 10% to 24% results in an increase of the release of indomethacin to practically quantitative release within 10 minutes.

Coating of cores with enteric coating

An enteric coating suspension was prepared by homogenizing 9.0 kg Eudragit® S 12.5 together with 0.135 kg acetyltributylcitrate, 0.9 kg talc and 7.965 kg isopropanol.

10 kg of the above-described cores containing 24% of talc were coated with 4.167 kg of this coating suspension in a fluidized bed, and the resulting pellets were covered with talc.

For the preparation of a pharmaceutical dosage form, more than 1000 of these pellets were filled into a capsule No. 1. Each capsule contained 75 mg indomethacin.

Example 2

The effect of dispersion-accelerating agents with respect to improving the dissolution of the active substance

In a similar manner as described in Example 1, (but without co-comminution of the indomethacin with any dispersion-accelerating agent), cores were prepared from 3.2 kg indomethacin, 1.0 kg microcrystalline cellulose, 5.7 kg sucrose powder and 0.1 kg polyvinylpyrrolidone. These cores are designated cores, type 0.

Another portion of cores, designated cores, type SACH, was prepared from the sme ingredients in a similar manner, except that in this case the indomethacin and the sucrose powder were co-comminuted by passage through a grinder using a 0.5 mm sieve.

In the same manner as described in Example 1, cores were made from 3.2 kg indomethacin, 0.2 kg sodium, laurylsulphate, 1.0 kg microcrystalline cellulose, 5.5 kg sucrose powder and 0.1 kg polyvinylpyrrolidone. These cores are designated cores, type NaLS.

The release of the indomethacin was measured, at pH 7.5 as described under Materials and Methods for these 3 types of cores.

The amount of indomethacin released at pH 7.5 after 10 minutes appears from Table II.

TABLE II

Percentage of indomethacin released at pH=7.5
after 10 min (n=2)

| | |
|---|---|
| Cores, type 0. | 71.0% |
| Cores, type SACH | 92.8% |
| Cores, type NaLS | 97.1% |

It appears from Table II that the release of indomethacin is considerably increased when a dispersion accelerating agent is co-comminuted with the indomethacin, and that the detergent type of dispersion-accelerating agent results in the fastest release.

Example 3
The influence of coatings soluble at different pH on the dissolution of indomethacin

An enteric coating suspension was prepared as described in Example 1 from 2.08 kg Eudragit® L12.5, 2.08 by Eudragit® S12.5, 0.0625 kg acetyltributylcitrate, 0.417 kg talc and 3.69 kg isopropanol.

This coating, which is soluble at pH above 6.5, was called Coating A.

An enteric coating suspension was prepared as described in Example 1 from 4.16 kg Eudragit® S12.5, 0.0625 kg acetyltributylcitrate, 0.417 kg talc and 3.69 kg isopropanol.

This coating, which is soluble at pH above 7.0, was called Coating B.

Cores containing sodium laurylsulphate and 24% of talc, prepared as described in Example 1, were coated with 10% of Coating A or 10% of Coating B (% dry matter of coating, calculated on the weight of the core). The dissolution of indomethacin from the resulting two types of pellets was determined as described under Materials and Methods. The results are stated in Table III.

TABLE III
Percentage of indomethacin released at pH 6.5 and pH 7.5 (n=3)

|  | pH=6.5 | | | pH=7.5 |
|---|---|---|---|---|
|  | 10 m | 30 m | 60 m | 60 m |
| Coating A | 17.4 | 64.0 | 76.5 | 98.6 |
| Coating B | 6.5 | 9.0 | 9.8 | 100.7 |

It appears from Table III that cores coated with Coating A and Coating B quantitatively released the indomethacin at pH 7.5 within 60 minutes and that cores coated with Coating B only released about 10% of indomethacin after 1 h at pH 6.5. The possibility of adjusting the enteric coating is very important because it makes it possible to tailor formulations to be disintegrated in a predetermined segment of the small intestine.

Example 4
Bioavailability of indomethacin from two multiple-units controlled release formulations
Drug formulations:

The two types of indomethacin-containing pellets prepared in Example 3 (designated Coating A and Coating B, respectively) were formulated into hard gelatin capsules designated Coating A and Coating B capsules, respectively. Each capsule of each formulation contained 75 mg indomethacin. Instant release capsules of indomethacin (Indocid®, Merck, Sharp and Dohme Ltd.) were used as the reference formulation. Each capsule of the reference formulation contained 25 mg indomethacin. Indomethacin was almost completely released from this capsule formulation during 10 minutes at pH 6.5.

Drug administration:

Eight healthy normal adult male subjects of an age range of 21—24 years and a body weight range of 60—80 kg were selected for this study.

Each subject fasted for 12 hours before drug administration and remained fasting for 4 hours afterwards. Administration was conducted in a three-way complete crossover with one week between dosing, in which each subject received orally one Coating A or B capsule or three capsules of the reference formulation (75 mg total dose) together with 100 ml water. Blood samples (10 ml) were withdrawn before dosing and at intervals during the following 24 hours.

Measurement of indomethacin in plasma:

Concentrations of indomethacin in plasma were measured using a high performance liquid chromatographic (HPLC) method. Plasma (200 μl for concentrations between 0.1 μg/ml and 4 μg/ml or 100 μl for concentrations above 4 μg/ml containing 1 μg mefenamic acid as an internal standard) was mixed with phosphate buffer (1 ml, 1 $M$, pH 5.0), and extracted with freshly distilled diethyl ether (5 ml) for 10 minutes on a rotary mixer. The phases were separated by centrifugation and the organic phase was removed and evaporated to dryness under nitrogen at 37°C. The residue was washed to the bottom of the tube with a small amount of ether which was then evaporated to dryness.

The drug residues were dissolved in methanol (50 μl), portions (20 μl) of which were injected into the HPLC system which consisted of an automatic injector and pump (Waters Associates Ltd., U.K.), fitted with a variable wavelength ultra-violet monitor (Pye Unicam Ltd., U.K.) operated at 260 nm (λmax for indomethacin in methanol). The stainless steel column (30 cm×0.4 cm i.d.) was prepacked with μ Bondapak

$C_{18}$ (mean particle size 10 µm, Waters Associates Ltd.) and a stainless steel precolumn (7 cm×0.2 cm i.d.) drypacked with pellicular CO: Pell® ODS (particular size 25—37 µm, Whatman Ltd., UK) was installed to protect the analytical column. Chromatography was performed in reversed-phase mode with a mobile phase of acetonitrile (62%, v/v) in phosphate buffer (0.1 $M$, pH 4.0) at a flow rate of 2.5 ml/min. Indomethacin and the internal standard (mefenamic acid) were eluted with retention times of 2.7 and 3.6 minutes respectively.

Linear calibration curves of peak area ratio of indomethacin to internal standard were constructed by analysis of plasma containing these compounds over the concentration range 0.1 µg/l—4 µg/l. The standard error of taking the calibration line as a measure of indomethacin concentration over this range was 0.12 µg/ml. The recovery of the internal standard at the level added of 5 µg/ml was 100%±4 S.D. (n=5), and the mean recovery of indomethacin over the concentration range 0.5 µg/l—4 µg/l was 103%±3 S.D. (n=5). No peaks were present on chromatograms of extracts of predose plasma in the position of the internal standard, but in some samples of predose plasma, interfering material was present at the position of indomethacin and equivalent to a maximum of 0.1 µg/ml. The limit of detection was therefore arbitrarily set at 0.1 µg/ml. The precision of measurement was assessed by the coefficients of variation of the means of replicate measurements (n=6) of ±17% at 0.1 µg/ml, ±2% at 2 µg/ml and ±4% at 4 µg/ml. Known metabolites of indomethacin did not interfere with the measurement of the unchanged drug above a limit of 0.1 µg/ml.

Data processing:

Areas to 24 h (AUC) under the plasma concentration-time curves were calculated by the trapezoidal rule. Since plasma drug levels of 24 h after dosing were close to the limit of detection, these areas were considered to be equivalent to areas to infinite time. Since drug administration was unbalanced with respect to the dosing sessions, AUCs, peak plasma levels and their times of occurrence, times to reach a plasma level of 1.0 µg/ml were subjected to analysis of variance by regression techniques. Overall formulation-related effects were examined by the F-test and formulation means were tested pair-wise by the method of least significant differences (Snedecor & Cochran, 1967).

Results:

Peaks of mean plasma concentrations of indomethacin of 4.9 µg/ml, 3.0 µg/ml and 2.3 µg/ml occurred after single oral doses of 75 mg of the reference formulation and the Coating A and B capsule formulations respectively and these peaks of mean levels occurred at 1 h, 2 h and 3 h respectively, vide Fig. 1.

Indomethacin was more slowly absorbed from both Coating A and B capsules than from the reference capsules, and was more slowly absorbed from the Coating B capsules than it was from the Coating A capsules.

The bioavailability parameters appear in Table IV. The differences between formulations within these parameters are highly significant except for the AUC.

TABLE IV

Mean values of bioavailability parameters of indomethacin after administration of the reference and coating A and B capsules, respectively, Standard deviations are in parentheses.

|  | Reference | Coating A | Coating B |
|---|---|---|---|
| Area (µg h/ml) | 12.2 (4.0) | 13.7 (4.3) | 11.8 (2.4) |
| Peak plasma concentration (µg/ml) | 5.5 (1.2) | 3.8 (1.2) | 2.9 (0.8) |
| Time of peak concentration (h) | 1.0 (0.3) | 2.1 (0.6) | 3.5 (0.9) |
| Time to 1 µg/ml[a] (h) | 0.4 (0.2) | 1.2 (0.3) | 2.4 (0.7) |

[a] Time after dosing required to reach a plasma concentration of 1 µg/ml, by interpolation.

These data imply a considerable slower absorption rate after administration of the Coating B capsules compared with Coating A capsules and the reference formulations. The extent of bioavailability, however, was similar after administration of each preparation.

Discussion:

Formulation of indomethacin as multiple-units controlled-release capsules comprising enteric-coated pellets of different sensitivity to an alkaline environment did not affect the extent of drug bioavailability, and drug absorption was slower after administration of these pellets when compared with the standard reference formulation. Rates of absorption were in the order, reference formulation>Coating A capsules>Coating B capsules (Table IV); thus it was demonstrated that these absorption rates are ranked in order of their observed dissolution rates *in vitro* (Table III).

10

The present formulation technique takes into account the transit time and distribution of the pellets throughout the gastrointestinal tract (Bechgaard & Ladefoged, 1978) and the characteristic of a strictly alkaline-dependent erosion of the coating of pellets. The data confirm that the drug release from these pellets *in vivo* was dependent on an alkaline pH and that dissolution probably occurred in the distal part of the gastrointestinal tract, where the pH is relatively high (pH 6.5—7.5) and less variable than that in the proximal small intestine, which factor is more important in the non-fasting state. This finding is further supported by the low observed standard deviations of the bioavailability parameters after administration of the Coating A and B capsules (Table IV). These standard deviations were of the same order of magnitude as those after administration of the standard reference formulation. Thus the present multiple-units controlled release formulations represent a reliable and reproducible source of indomethacin.

Example 5
The effect of food on the bioavailability of indomethacin from a multiple-units controlled release formulation
Drug formulation:
Coating B capsules each containing 75 mg indomethacin, as described in Example 4.

Drug administration:
Nine healthy adult male subjects of an age range of 22—36 years and a body weight range of 63—70 kg, were selected for this study.
Administration was conducted as a complete crossover with one week between doses, in which each subject received a single oral dose of one capsule (75 mg) together with 100 ml water, once after a 12-hour fast, and once after they had received a breakfast consisting of cereal, egg, bacon and sausage, one slice of toast and one cup of coffee, within 15 minutes of drug administration. Blood samples were withdrawn before dosing, and at intervals during the following 24 hours.

Measurement of indomethacin in plasma:
Concentrations of indomethacin in plasma were measured by a high performance liquid chromatographic method, as described in Example 4.

Data processing:
Areas to 24 h (AUC) under the plasma concentration-time curves were calculated by the trapezoidal rule. Peak plasma concentrations and times of their occurrence, AUC, and times to reach a plasma concentrations of 1 µg/ml were subjected to analyses of variance for cross-over designs (Snedecor & Cochran, 1967), with subjects, dosing sessions, treatments and residual as factors in the analysis. The statistical significance of treatment differences was tested by the method of least significant differences.

Results:
A peak of mean concentrations of indomethacin in plasma of 1.9 µg/ml occurred at 5 hours after administration of 75 mg after a 12 h fast, and indomethacin was present (0.2 µg/ml) in plasma withdrawn 24 h after dosing. When 75 mg was administered within 15 min of ingestion of a substantial breakfast, the peak of mean plasma concentrations of indomethacin (1.8 µg/ml) occurred at 6 h after which mean plasma indomethacin concentrations declined to 0.4 µg/ml at 24 hours, *vide* Fig. 2.
Two peak levels of indomethacin concentrations were present in the plasma of most subjects upon administration after a period of fasting as well as together with food, but this effect was more noticeable when the doses were administered together with food.
Indomethacin is thought to undergo enterohepatic recirculation in humans, and the secondary peak plasma concentrations may have been an expression of this recirculation.
The overall major peak plasma concentrations and AUC were not significantly different (P>0.05) after administration of Coating B capsules either after a twelve-hour fast or together with food (Table V).

11

TABLE V
Mean bioavailability parameters of indomethacin. Standard deviations in parentheses.
(n=9)

| | Fasting | | With food ingestion | | |
|---|---|---|---|---|---|
| Area (µg.h/ml) | 13.8 | (3.8) | 12.5 | (2.6) | NS |
| Peak level (ng/ml): | | | | | |
| First | 2.7 | (0.8) | 2.2 | (1.0) | NS |
| Second | 0.5[a] | (0.2) | 1.1[b] | (0.8) | NS |
| Time of peak level (h): | | | | | |
| First | 4.2 | (1.4) | 6.4 | (2.2) | P<0.05 |
| Second | 12.7 | (1.0) | 14.4 | (6.8) | NS |
| Time (h) to reach 1 µg/ml[a] | 3.0 | (1.3) | 5.5 | (2.6) | P<0.05 |

[a] Secondary peak concentrations were present in the plasma of 6 subjects.
[b] Secondary peak concentrations were present in the plasma of 7 subjects.
Significance levels refer to treatment differences from the analysis of variance. NS=not significant (P>0.05).

The time of occurrence of the first peak plasma level after administration together with food (6.4 h) was later than, and significantly different from (P<0.05), that after administration after a twelve-hour fast (4.2 h), but corresponding times of occurrence of the second peak levels were not statistically significantly different. The time required to reach a plasma concentration of 1 µg/ml after administration together with food (5.5 h) was longer than, and significantly different from (P<0.05), that after administration after fasting (3.0 h), as seen from Table V.

Discussion:
Administration of Coating B capsules with food did not affect the extent of drug bioavailability, but the presence of food decreased the rate of bioavailability as indicated by the later, and statistically significantly different, time of occurrence of the first peak plasma concentration and the time to achieve a plasma concentration of 1 µg/ml. The phenomenon of the double peak concentration was also exaggerated after administration with food. Apparently, the extent to which a concomitant meal influences the bioavailability of indomethacin from Coating B capsules is equal to that from a plain indomethacin capsule. It should be emphasized that the observed standard deviations of the bioavailability parameters were of the same order of magnitude when the drug was administered together with food or after a fast, as seen from Table V. Thus, the controlled release multiple-units formulation according to the invention represents a reliable and reproducible source of indomethacin when administered together with food.

Example 6
Preparation of furosemide-containing cores to be coated with an enteric coating
Cores were prepared from 40 g of furosemide, 10 g of sucrose powder, 10 g of microcrystalline cellulose, 25 g of sucrose powder and 15 g of talc.
The furosemide and 10 g of the sucrose were passed through a grinder using a 0.5 mm sieve.
The powder was mixed with the microcrystalline cellulose, the remainder of the sucrose and the talc in a planet mixer.
100 g of the resulting mixture was moistened with 12 g purified water and was mixed until the mixture was granular.
The moist mixture was extruded through a 0.5 mm sieve.
The resulting extrudate was formed into compact-shaped cores in a marumerizer, and the cores were dried in a fluidized bed, the dried cores were sieved, the upper sieve being 0.71 mm, and the bottom sieve 0.46 mm.

Coating of cores with enteric coating
An enteric coating suspension (C) was prepared by homogenizing 11.4 g Eudragit® L 30 D together with 0.6 g triacetin and 8 g purified water.
Another enteric coating suspension (D) was prepared by homogenizing 25.0 g Eudragit® S 12.5 together with 0.375 g acetyltributylcitrate, 2.5 g talc and 22.1 g isopropanol.
Portions of each 100 g of the cores obtained above were coated with coating suspension C and D, respectively, in a fluidized bed, and the resulting pellets were covered with talc.

# 0 080 341

The release of furosemide from the resulting pellets was determined as described under Materials and Methods. The results are stated in Table VI.

TABLE VI
Percentage of furosemide released at pH 4.5
and at pH 7.5 (n=2)

| | pH 4.5 | pH 7.5 |
|---|---|---|
| | 120 m | 30 m |
| Coating C | 16.9 | 95.4 |
| Coating D | 14.3 | 96.5 |

It appears from Table VI that the release of furosemide is practically quantitative at pH 7.5, and that the furosemide is released very slowly at pH 4.5.

Literature

GB Patent 1 468 172

Eur. Patent Application 79 850 110, Publication 0 013 262

US Patent 4 193 985

Baggensen S, Bechgaard H, & Schmidt K (1981) Content and dissolution uniformity testing of controlled-release products: The Repro-Dose® quality control procedure. *Pharm. Acta Helv 56*, 85—92.

Bechgaard, H & Hegermann Nielsen, G (1978) Controlled release multiple-units and single-units doses. A literature review. *Drug Develop Ind Pharm 4*, 53—67.

Bechgaard, H & Ladefoged, K (1978) Distribution of pellets in the gastrointestinal tract. The influence on transit time exerted by the density or diameter of pellets. *J Pharm Pharmacol 30*, 690—692.

Bechgaard, H & Baggesen, S (1980) Propoxyphene and norpropoxyphene: Influence of type of controlled release formulation on intra- and intersubject variations. *J Pharm Sci 69*, 1327—1330.

Bogentoft, C, Carlsson, I. Ekenved, G & Magnusson, A (1978) Influence of food on the absorption of acetylsalicylic acid from enteric-coated dosage forms. *Eur J Clin Pharmacol 14*, 351—355.

Green, DM (1966) Tablets of coated aspirin microspherules—A new dosage form. *J New Drugs 6*, 294—303.

McDonald, PJ, Mather, LE & Story, MJ (1977) Studies on absorption of a newly developed enteric-coated erythromcyin base. *J Clin Pharmacol 17*, 601—606.

Snedecor, GW & Cochran, WG (1967) Statistical Methods, Iowa State University Press, Iowa, 271—275.

**Claims for the Contracting States: BE, CH/LI, DE, FR, GB, IT, LU, NL, SE**

1. A pharmaceutical oral controlled release multiple-units formulation which comprises particles comprising an active substance requiring more than 30 parts by volume of water to dissolve 1 part by weight of the active substance at ambient temperature, in intimate admixture with a dispersion-accelerating substance which is readily soluble in intestinal fluids, which particles are combined into cross-sectionally substantially homogeneous cores together with at least one component which enhances the disintegration of the cores in intestinal fluids, the cores being coated with a coating which is substantially resistant to gastric conditions, but which is erodable under the conditions prevailing in the small intestine.

2. A formulation according to claim 1 in which the active substance is a substance which requires more than 1000 parts by volume of water to dissolve 1 part by weight of the active substance at ambient temperature.

3. A formulation according to claim 1 or 2 in which the active substance is selected from indomethacin, spironolactone, ibuprofen, furosemide, sulfadiazine, sulfamerazine, progesterone, reserpine, pyrvinium embonate, mofebutazone, hydrochlorothiazide, tetracycline, tolbutamide, acetaminophen, testosterone, valproic acid, estradiol, acetazolamide, erythromycin, iron salts, hydralazine, carbamazepine, quinidine, and cardiac glycosides, e.g., digoxin.

4. A formulation according to claim 1 in which the component which enhances the disintegration of the cores in intestinal fluids is selected from particulate substances which are insoluble in gastric and intestinal fluids.

5. A formulation according to claim 4 in which the particulate substances are selected from plate-shaped particles, e.g. talc, and/or compact-shaped particles, e.g. aluminium silicate, zinc oxide, magnesium oxide, titanium dioxide, colloidal silica or magnesium trisilicate.

6. A formulation according to claim 1 in which the disintegration-enhancing component comprises particles of a substance which is readily soluble in intestinal fluids, e.g. sucrose, glucose, mannitol, sorbitol or lactose.

7. A formulation according to claims 5 and 6 in which the disintegration-enhancing components comprise a combination of talc and sucrose.

8. A formulation according to claim 1 in which the particles consisting of an intimate admixture of the active substance and the dispersion-accelerating substance have a size of 1—10 µm, in particular 2—5 µm.

9. A formulation according to claim 8 in which the dispersion-accelerating substance is a surface-active substance.

10. A formulation according to claim 9 in which the particles contain the surface-active substance in an amount of not more than 10% by weight of the active substance.

11. A formulation according to claim 9 in which the surface-active substance is an anionic or non-ionic detergent.

12. A formulation according to claim 11 in which the surface-active substance is selected from sodium salts of fatty alcohol sulphates, sulphosuccinates, partial fatty acid esters of sorbitans, e.g. sorbitanmonooleate, partial fatty acid esters of polyhydroxyethylene sorbitans, e.g. polyethylene glycol sorbitan monooleate, or polyhydroxyethylene fatty alcohol ethers, e.g. polyhydroxyethylene (23) lauryl ether.

13. A formulation according to claim 12 in which the surface-active substance is sodium lauryl sulphate.

14. A formulation according to claim 1 in which the erodable coating is an enteric coating.

15. A formulation according to claim 14 in which the enteric coating is selected from acrylic polymers and cooplymers, e.g. a polymerisate of methacrylic acid and methacrylic acid methyl ester, shellac, cellulose acetate esters such as mixed partial esters of cellulose containing phthalate groups, acetyl groups and free acid groups (cellulose acetate phthalate), polyvinyl acetate esters such as polyvinyl acetate phthalate, hydroxypropylmethyl cellulose esters such as hydroxypropylmethylcellulose phthalate, or alkyleneglycol ether esters of copolymers such as partial ethyleneglycol monomethyl ether ester of ethyl acrylate-maleic anhydride copolymer, propyleneglycol monomethyl ether ester of ethyl acrylate-maleic anhydride copolymer, dipropyleneglycol monomethyl ether ester of ethyl acrylate-maleic anhydride copolymer or diethyleneglycol monomethyl ether ester of methyl acrylate-maleic anhydride copolymer, n-butyl acrylate-maleic anhydride copolymer, isobutylacrylate-maleic anhydride copolymer or ethylacrylate-maleic anhydride copolymer.

16. A formulation according to claim 15 wherein the coating is a coating which is selectively eroded in the distal small instestine.

17. A formulation according to claim 16 wherein the enteric coating is substantially insoluble at a pH below 7.

18. A formulation according to claim 17 in which the enteric coating is a coating which will release at least 90% of the active substance within one hour in 250 ml of a 0.05M phosphate buffer, pH 7.5 at 37±0.1°C and a rotation speed of 30±1 r.p.m.

19. A formulation according to claim 17 or 18 in which the enteric coating comprises an acrylic polymer.

20. A formulation according to claim 19 in which the enteric coating comprises a polymerisate of methacrylic acid and methacrylic acid methyl ester or mixtures thereof.

21. A formulation according to claim 1 in which the diameter of each coated core is 0.4—1.2 mm, in particular 0.5—1.0 mm, e.g. 0.5—0.7 mm.

22. A formulation according to claim 21 in which the amount of enteric coating is 2—25% by weight of dry matter, calculated on the total weight of the cores.

23. A formulation according to claim 22 in which each coated core has a diameter of 0.5—0.6 mm, and the amount of enteric coating applied is 4—12% by weight of dry matter, calculated on the total weight of the cores.

24. A method of preparing a pharmaceutical oral controlled release multiple-units preparation or units therefor, in which an active substance requiring more than 30 parts by volume of water to dissolve 1 part by weight of the active substance at ambient temperature is comminuted together with a dispersion-accelerating substance which is readily soluble in intestinal fluids to obtain particles in which the active substance is intimately admixed with the dispersion-accelerating substance, the particles are combined into cross-sectionally substantially homogeneous cores together with at least one component which enhances the disintegration of the cores in intestinal fluids, the cores are coated with a coating which is substantially resistant to gastric conditions, but which is erodable under the conditions prevailing in the small intestine, and if desired a multiplicity of the coated cores is combined into a capsule or tablet formulation.

**Claims for the Contracting State: AT:**

1. A method of preparing a pharmaceutical oral controlled release multiple-units preparation or units therefor, in which an active substance requiring more than 30 parts by volume of water to dissolve 1 part by weight of the active substance at ambient temperature is comminuted together with a dispersion-

14

accelerating substance which is readily soluble in intestinal fluids to obtain particles in which the active substance is intimately admixed with the dispersion-accelerating substance, the particles are combined into cross-sectionally substantially homogeneous cores together with at least one component which enhances the disintegration of the cores in intestinal fluids, the cores are coated with a coating which is substantially resistant to gastric conditions, but which is erodable under the conditions prevailing in the small intestine, and if desired a multiplicity of the coated cores is combined into a capsule or tablet formulation.

2. A method according to claim 1 in which the active substance is a substance wihch requires more than 1000 parts by volume of water to dissolve 1 part by weight of the active substance at ambient temperature.

3. A method according to claim 1 or 2 in which the active substance is selected from indomethacin, spironolactone, ibuprofen, furosemide, sulfadiazine, sulfamerazine, progesterone, reserpine, pyrvinium embonate, mofebutazone, hydrochlorothiazide, tetracycline, tolbutamide, acetaminophen, testosterone, valproic acid, estradiol, acetazolamide, erythromycin, iron salts, hydralazine, carbamazeoine, quinidine, and carbiac glycosides, e.g. digoxin.

4. A method according to claim 1 in which the component which enhances the disintegration of the cores in intestinal fluids is selected from particulate substances which are insoluble in gastric and intestinal fluids.

5. A method according to claim 4 in which the particulate substances are selected from plate-shaped particles, e.g. talc, and/or compact-shaped particles, e.g. aluminium silicate, zinc oxide, magnesium oxide, titanium dioxide, colloidal silica or magnesium trisilicate.

6. A method according to claim 1 in which the disintegration-enhancing component comprises particles of a substance which is readily soluble in intestinal fluids, e.g. sucrose, glucose, mannitol, sorbitol or lactose.

7. A method according to claims 5 and 6 in which the disintegration-enhancing components comprise a combination of talc and sucrose.

8. A method according to claim 1 in which the particles consisting of an intimate admixture of the active substance and the dispersion-accelerating substance have a size of 1—10 μm, in particular 2—5 μm.

9. A method according to claim 8 in which the dispersion-accelerating substance is a surface-active substance.

10. A method according to claim 9 in which the particles contain the surface-active substance in an amount of not more than 10% by weight of the active substance.

11. A method according to claim 9 in which the surface-active substance is an anionic or non-ionic detergent.

12. A method according to claim 11 in which the surface-active substance is selected from sodium salts of fatty alcohol sulphates, sulphosuccinates, partial fatty acid esters of sorbitans, e.g. sorbitanmonooleate, partial fatty acid esters of polyhydroxyethylene sorbitans, e.g. polyethylene glycol sorbitan monooleate, or polyhydroxyethylene fatty alcohol ethers, e.g. polyhydroxyethylene (23) lauryl ether.

13. A method according to claim 12 in which the surface-active substance is sodium lauryl sulphate.

14. A method according to claim 1 in which the erodable coating is an enteric coating.

15. A method according to claim 14 in which the enteric coating is selected from acrylic polymers and copolymers, e.g. a polymerisate of methacrylic acid and methacrylic acid methyl ester, shellac, cellulose acetate esters such as mixed partial esters of cellulose containing phthalate groups, acetyl groups and free acid groups (cellulose acetate phthalate), polyvinyl acetate esters such as polyvinyl acetate phthalate, hydroxypropylmethyl cellulose esters such as hydroxypropylmethyl cellulose phthalate, or alkyleneglycol ether esters of copolymers such as partial ethyleneglycol monomethyl ether ester of ethyl acrylate-maleic anhydride copolymer, propyleneglycol monomethyl ether ester of ethyl acrylate-maleic anhydride copolymer, dipropyleneglycol monomethyl ether ester of ethyl acrylate-maleic anhydride copolymer or diethyleneglycol monomethyl ether ester of methyl acrylate-maleic anhydride copolymer, n-butyl acrylate-maleic anhydride copolymer, isobutylacrylate-maleic anhydride copolymer or ethylacrylate-maleic anhydride copolymer.

16. A method according to claim 15 wherein the coating is a coating which is selectively eroded in the distal small intestine.

17. A method according to claim 16 wherein the enteric coating is substantially insoluble at a pH below 7.

18. A method according to claim 17 in which the enteric coating is a coating which will release at least 90% of the active substance within one hour in 250 ml of a 0.05M phosphate buffer, pH 7.5, at 37±0.1°C and a rotation speed of 30±1 r.p.m.

19. A method according to claim 17 or 18 in which the enteric coating comprises an acrylic polymer.

20. A method according to claim 19 in which the enteric coating comprises a polymerisate of methacrylic acid and methacrylic acid methyl ester or mixtures thereof.

21. A method according to claim 1 in which the diameter of each coated core is 0.4—1.2 mm, in particular 0.5—1.0 mm, e.g. 0.5—0.7 mm.

22. A method according to claim 21 in which the amount of enteric coating is 2—25% by weight of dry matter, calculated on the total weight of the cores.

23. A method according to claim 22 in which each coated core has a diameter of 0.5—0.6 mm, and the amount of enteric coating applied is 4—12% by weight of dry matter, calculated on the total weight of the cores.

**Patentansprüche für die Vertragsstaaten: BE, CH, DE, FR, GB, IT, LI, LU, NL, SE**

1. Pharmazeutische orale, aus mehreren Einheiten bestehende Formulierung mit gesteuerter Freisetzung, dadurch gekennzeichnet, dass sie Einheiten enthält, die eine aktive Substanz enthalten, welche Substanz mehr als 30 Volumenteile Wasser benotigt, um ein Gewichtsteil der aktiven Substanz bei Umgebungstemperatur aufzulösen, in innigem Gemisch mit einer dispergierungsbeschleunigenden Substanz, die in Darmflüssigkeiten leicht löslich ist, welche Einheiten zu im Querschnitt im wesentlichen homogenen Kernen kombiniert sind, zusammen mit mindestens einem Bestandteil, der die Zersetzung der Kerne in Darmflüssigkeiten beschleunigt, wobei die Kerne mit einer Beschichtung beschichtet sind, die im wesentlichen gegen im Magen herrschende Bedingungen beständig ist, jedoch unter den im Dünndarm herrschende Bedingungen erodiert werden kann.

2. Formulierung nach Anspruch 1, dadurch gekennzeichnet, dass die aktive Substanz eine Substanz ist, die mehr als 1000 volumenteile Wasser benögtigt, um ein Gewichtsteil der aktiven Substanz bei Umgebungstemperatur aufzulösen.

3. Formulierung nach Anspruch 1 oder 2, dadurch gekennzeichnet, dass die aktive Substanz aus der folgenden Gruppe ausgewählt ist, Indomethacin, Spironolacton, Ibuprofin, Furosemid, Sulfadiazin, Sulfamerazin, Progesteron, Reserpin, Pyrvinium-embonat, Mofebutazon, Hydrochlorothiazid, Tetracyclin, Tolbutamin, Acetaminophen, Testosteron, Valproesäure, Estradiol, Acetazolamid, Erythromycin, Eisensalze, Hydralazin, Carbamazepin, Chinizidin und Herzglykoside, z.B. Digoxin.

4. Formulierung nach Anspruch 1, dadurch gekennzeichnet, dass der Bestandteil, der die Zersetzung der Kerne in Darmflüssigkeiten beschleunigt, aus Teilchen, die in Magen- und Darmflüssigkeiten unlöslich sind, ausgewählt ist.

5. Formulierung nach Anspruch 4, dadurch gekennzeichnet, dass die Teilchen aus plattenförmigen Körper, z.B. Talk, und, oder Körper kompakter Form, z.B. Aluminiumsilicat, Zinkoxid, Magnesiumoxid, Titandioxid, kolloidalem Siliciumdioxid oder Magnesiumtrisilicat, ausgewählt sind.

6. Formulierung nach Anspruch 1, dadurch gekennzeichnet, dass der die Zersetzung beschleunigenden Bastandteil Teilchen einer Substanz enthält, die in Darmflüssigkeiten leicht löslich ist, wie z.B. Saccharose, Glucose, Mannit, Sorbit oder Lactose.

7. Formulierung nach Anspruch 5 oder 6, dadurch gekennzeichnet, dass die die Zersetzung beschleunigenden Bestandteile eine Kombination aus Talk und Saccharose umfassen.

8. Formulierung nach Anspruch 1, dadurch gekennzeichnet, dass die Teilchen, die aus einem innigen Gemisch der aktiven Substanz und der dispergierungsbeschleunigenden Substanz bestehen, eine Größe von 1—10 µm, insbesondere 2—5 µm, haben.

9. Formulierung nach Anspruch 8, dadurch gekennzeichnet, dass die dispergierungsbeschleunigende Substanz eine oberflächenaktive Substanz ist.

10. Formulierung nach Anspruch 9, dadurch gekennzeichnet, dass die Teilchen die oberflächenaktive Substanz in einer Menge von höchstens 10 Gewichtsprozent, bezogen auf die aktive Substanz, enthalten.

11. Formulierung nach Anspruch 9, dadurch gekennzeichnet, dass die oberflächenaktive Substanz eine anionische oder nicht-ionische Detergens ist.

12. Formulierung nach Anspruch 11, dadurch gekennzeichnet, dass die oberflächenaktive Substanz aus der folgenden Gruppe ausgewählt ist, Natriumsalze von Fettalkoholsulphaten, Sulphosuccinate, Fettsäureteilester von Sorbitanen, z.B. Sorbitanmonooleat, Fettsäureteilester von Polyhydroxyethylensorbitanen, z.B. Polyethylenglycolsorbitanmonooleat, oder Polyhydroxyethylenfettalkoholether, z.B. Polyhydroxyethylen-(23)-Laurylether.

13. Formulierung nach Anspruch 12, dadurch gekennzeichnet, dass die oberflächenaktive Substanz Natriumlaurylsulphat ist.

14. Formulierung nach Anspruch 1, dadurch gekennzeichnet, dass die erodierbare Beschichtung eine enterische Beschichtung ist.

15. Formulierung nach Anspruch 14, dadurch gekennzeichnet, dass die enterische Beschichtung aus der folgenden Gruppe gewählt ist: Acrylpolymere und Mischpolymere, z.B. ein Polymerisat von Methacrylsäure und Methacrylsäureester, Schellack, Zelluloseacetatester, wie z.B. gemischte Teilester von Zellulose, die Phthalatgruppen, Acetylgruppen und freie Säuregruppen enthalten (Zelluloseacetatphthalat), Polyvinylacetatester, wie z.B. Polyvinylacetatphthalat, Hydroxypropylmethylzelluloseester, wie z.B. Hydroxypropylmethylzellulosephthalat oder Alkylenglykolether-ester von Mischpolymeren wie z.B. Ethylenglykolmonomethylether - Teilester von Ethylacrylat - Maleinsäureanhydrid - Mischpolymerisat, Propylenglykolmonomethylether - ester von Ethylacrylat - Maleinsäureanhydrid - Mischpolymerisat, Dipropylenglykolmonomethylether - ester von Ethylacrylat - Maleinsäureanhydrid - Mischpolymerisat, Butylacrylat - Maleinsäureanhydrid - Mischpolymerisat, Isobutylacrylat - Maleinsäureanhydrid - Mischpolymerisat oder Ethylacrylat-Maleinsäureanhydrid-Mischpolymerisat.

16. Formulierung nach Anspruch 15, dadurch gekennzeichnet, dass die Beschichtung eine Beschichtung ist, die selektiv im distalen Teil des Dünndarms erodiert wird.

17. Formulierung nach Anspruch 16, dadurch gekennzeichnet, dass die enterische Beschichtung im wesentlichen bei einem pH unter 7 unlöslich ist.

18. Formulierung nach Anspruch 17, dadurch gekennzeichnet, dass die enterische Beschichtung eine Beschichtung ist, die wenigstens 90% der aktiven Substanz innerhalb einer Stunde in 250 ml einer 0,05 Phosphatpuffer, pH 7,5, bei 37±0,1°C und bei einer Rotationsgeschwindigkeit von 30±1 Drehungen/Minute freisetzt.

19. Formulierung nach Anspruch 17 oder 18, dadurch gekennzeichnet, dass die enterische Beschichtung ein Acrylpolymerisat enthält.

20. Formulierung nach Anspruch 19, dadurch gekennzeichnet, dass die enterische Beschichtung ein anionisches Polymerisat von Methacrylsäure und Methacrylsäuremethylester oder Mische hiervon enthält.

21. Formulierung nach Anspruch 1, dadurch gekennzeichnet, dass der Durchmesser jedes beschichteten Kerns 0,4—1,2 mm, insbesondere 0,5—1,0 mm, z.B. 0,5—0,7 mm, beträgt.

22. Formulierung nach Anspruch 21, dadurch gekennzeichnet, dass der Trockengehalt der enterischen Beschichtung 2—25 Gewichtsprozent, bezogen auf das Gesamtgewicht der Kernen, beträgt.

23. Formulierung nach Anspruch 22, dadurch gekennzeichnet, dass der Durchmesser jedes beschichteten Kerns 0,5—0,6 mm und der Trockengewicht der enterischen Beschichtung 4—12 Gewichtsprozent, bezogen auf das Gesamtgewicht der Kernen, beträgt.

24. Verfahren zur Herstellung einer pharmazeutischen oralen, aus mehreren Einheiten bestehenden Formulierung mit gesteuerter Freisetzung oder Einheiten dafür, dadurch gekennzeichnet, dass eine aktive Substanz, die mehr als 30 Volumenteile Wasser benötigt, um ein Gewichtsteil der aktiven Substanz bei Umgebungstemperatur aufzulösen, zusammen mit einer dispergierungsbeschleunigenden Substanz, die in Darmflüssigkeiten leicht löslich ist, feingemahlen wird, um Einheiten zu erhalten, worin die aktive Substanz in innigem Gemisch mit der dispergierungsbeschleunigenden Substanz ist, dass die Einheiten zu im Querschnitt im wesentlichen homogenen Kernen kombiniert werden, zusammen mit mindestens einem Bestandteil, der die Zersetzung der Kerne in Darmflüssigkeiten beschleunigt, dass die Kerne mit einer Beschichtung beschichtet werden, die im wesentlichen gegen im Magen herrschende Bedingungen beständig ist, jedoch unter den im Dünndarm herrschenden Bedingungen erodiert werden kann, und dass eine Vielfaltigkeit der beschichteten Kernen gewünschterfalls zu einer Kapsel- oder Tablettformulierung kombiniert wird.

**Patentansprüche für den Vertragsstaat: AT**

1. Verfahren zur Herstellung einer pharmazeutischen oralen aus mehreren Einheiten bestehenden Formulierung mit gesteuerter Frisetzung oder Einheiten hierfür, dadurch gekennzeichnet, daß man eine aktive Substanz, die mehr als 30 Volumenteile Wasser benötigt, um einen Gewichtteil der aktiven Substanz bei Umgebungstemperatur aufzulösen, zusammen mit einer dispergierungsbeschleunigenden Substanz, die leicht löslich in Darmflüssigkeiten ist, zerreibt, um Teilchen zu erhalten, die aktive Substanz in inniger Vermischung mit der dispergierungsbeschleunigenden Substanz enthält, die erhaltenden Teilchen zu Kernen mit im wesentlichen homogenen Querschnitt kombiniert zusammen mit mindestens einem Bestandteil, der die Zersetzung der Kerne in Darmflüssigkeiten beschleunigt, die einzelne Kerne mit einer Beschichtung, die im wesentlichen gegen im Magen herrschenden Bedingungen beständig ist, jedoch unter den im Dünndarm herrschenden Bedingungen erodiert werden kann, und, falls gewünscht, eine Vielzahl der beschichteten Kerne in einer Kapsel- oder Tablettenformulierung kombiniert.

2. Verfahren nach Anspruch 1, dadurch gekennzeichnet, daß die aktive Substanz eine Substanz ist, die mehr als 1000 Volumenteile Wasser benötigt, um einen Gewichtteil der aktiven Substanz bei Umgebungstemperatur aufzulosen.

3. Verfahren nach Anspruch 1 oder 2, dadurch gekennzeichnet, daß die aktive Substanz aus der folgenden Gruppe ausgewählt ist Indomethacin, Spironolacton, Ibuprofen, Furosemid, Sulfadiazin, Sulfamerazin, Progesteron, Reserpin, Pyrviniumembonat, Mofebutazon, Hydrochlorothazid, Tetracyclin, Tolbutamid, Acetaminophen, Testosteron, Vapron, Estradiol, Acetazolamid, Erthromycin, Eisensalze, Hydralazin, Carbamazepin, Quinidin und Herzglucoside, z.B. Digoxin.

4. Verfahren nach Anspruch 1, dadurch gekennzeichnet, daß der Bestandteil, der die Zersetzung der Kerne in Darmflüssigkeiten beschleunigt, aus partikelförmigen Substanzen, die in Magen- und Darmflüssigkeiten nicht löslich sind, gewählt ist.

5. Verfahren nach Anspruch 4, dadurch gekennzeichnet, daß die partikelförmigen Substanzen aus plattenförmigen Körpern, z.B. Talk, und/oder Körper kompakter Form, z.B. Aluminiumsilikat, Zinkoxid, Magnesiumoxid, Titandioxid, kolloidalem Siliciumdioxid oder Magnesiumtrisilikat gewählt sind.

6. Verfahren nach Anspruch 1, dadurch gekennzeichnet, daß der Bestandteil, der die Zersetzung beschleunigt, Teilchen einer Substanz, die in Darmflüssigkeiten leicht löslich ist, z.B. Saccharose, Glucose, Mannitol, Sorbitol oder Lactose, umfasst.

7. Verfahren nach Anspruch 5 oder 6, dadurch gekennzeichnet, daß die Bestandteile, die die Zersetzung beschleunigen, eine Kombination aus Talk und Saccharose umfassen.

8. Verfahren nach Anspruch 1, dadurch gekennzeichnet, daß die Teilchen, die aus einem innigen

# 0 080 341

Gemisch von einer aktiven Substanz und einer dispergierungsbeschleunigenden Substanz bestehen, eine Größe von 1—10 µm, insbesondere 2—5 µm, haben.

9. Verfahren nach Anspruch 8, dadurch gekennzeichnet, daß die dispergierungsbeschleunigende Substanz eine oberflächenaktive Substanz ist.

10. Verfahren nach Anspruch 9, dadurch gekennzeichnet, daß die Teilchen die oberflächenaktive Substanz in einer Menge von höchstens 10 Gewichtsprozent bezogen auf die aktive Substanz, enthälten.

11. Verfahren nach Anspruch 9, dadurch gekennzeichnet, daß die oberflächenaktive Substanz eine anionische oder nicht-ionische Detergens ist.

12. Verfahren nach Anspruch 11, dadurch gekennzeichnet, daß die oberflächenaktive Substanz aus der folgenden Gruppe ausgewählt ist, Natriumsalze von Fettalkoholsulphaten, Sulphosuccinate, Fettsäureteilester von Sorbitanen, z.B. Sorbitanmonooleat, Fettsäureteilester von Polyhydroxyethylensorbitanen, z.B. Polyethylenglycolsorbitanenmonooleat oder Polyhydroxyethylenfettalkoholether, z.B. Polyhydroxyethylen-(23)-Laurylether.

13. Verfahren nach Anspruch 12, dadurch gekennzeichnet, daß die oberflächenaktive Substanz Natriumlaurylsulphat ist.

14. Verfahren nach Anspruch 1, dadurch gekennzeichnet, daß die erodierbare Beschichtung eine enterische Beschichtung ist.

15. Verfahren nach Anspruch 14, dadurch gekennzeichnet, daß die enterische Beschichtung aus der folgenden Gruppe gewählt ist: Acrylpolymerisate und Mischpolymerisate, z.B. ein Polymerisat von Methacrylsäure und Methacrylsäureester, Schellack, Zelluloseacetatester, wie z.B. gemischte Teilester von Zellulose, die Phthalatgruppen, Acetylgruppen und freie Säuregruppen enthalten (Zelluloseacetatphthalat), Polyvinylacetatester, wie z.B. Polyvinylacetatphthalat, Hydroxypropylmethylzelluloseester, wie z.B. Hydroxypropylmethylzellulosephthalat oder Alkylenglykolether-ester von Mischpolymeren wie z.B. Ethylenglykolmonomethylether - Teilester von Ethylacrylat - Maleinsäureanhydrid - Mischpolymerisat, Propylenglykolmonomethylether - ester von Ethylacrelat - Maleinsäureanhydrid - Mischpolymerisat, Dipropylenglykolmonomethylether - ester von Ethylacrylat - Maleinsäureanhydrid - Mischpolymerisat, Butylacrylat - Maleinsäureanhydrid - Mischpolymerisat, Isobutylacrylat - Maleinsäureanhydrid - Mischpolymerisat oder Ethylacrylat-Maleinsäureanhydrid-Mischpolymerisat.

16. Verfahren nach Anspruch 15, dadurch gekennzeichnet, daß die Beschichtung eine Beschichtung ist, die selektiv im distalen Teil des Dunndarms erodiert wird.

17. Verfahren nach Anspruch 16, dadurch gekennzeichnet, daß die enterische Beschichtung im wesentlichen bei einem pH unter 7 unloslich ist.

18. Verfahren nach Anspruch 17, dadurch gekennzeichnet, daß die enterische Beschichtung eine Beschichtung ist, die wenigstens 90% der aktiven Substanz innerhalb einer Stunde in 250 ml einer 0,05 Phosphatpuffer, pH 7,5, bei 37±0,1°C und bei einer Rotationsgeschwindigkeit von 30±1 Drehungen/Minute freisetzt.

19. Verfahren nach Anspruch 17 oder 18, dadurch gekennzeichnet, daß die enterische Beschichtung ein Acrylpolymerisat enthalt.

20. Verfahren nach Anspruch 19, dadurch gekennzeichnet, daß die enterische Beschichtung ein anionisches Polymerisat von Methacrylsäure und Methacrylsäuremethylester oder Mische hiervon enthält.

21. Verfahren nach Anspruch 1, dadurch gekennzeichnet, daß der Durchmesser jedes beschichteten Kerns 0,4—1,2 mm, insbesondere 0,5—1,0 mm, z.B. 0,5—0,7 mm, beträgt.

22. Verfahren nach Anspruch 21, dadurch gekennzeichnet, daß der Trockengehalt der enterischen Beschichtung 2—25 Gewichtsprozent, bezogen auf das Gesamtgewicht der Kernen, beträgt.

23. Verfahren nach Anspruch 22, dadurch gekennzeichnet, daß der Durchmesser jedes beschichteten Kerns 0,5—0,6 mm und der Trockengewicht der enterischen Beschichtung 4—12 Gewichtsprozent, bezogen auf das Gesamtgewicht der Kernen, beträgt.

**Revendications pour les Etats Contractants: BE, CH, DE, FR, GB, IT, LI, LU, NL, SE**

1. Une formulation pharmaceutique à unités multiples à libération contrôles pour l'administration par voie orale, quelle formulation comprend des particules comprenant une substance active qui exige plus de 30 parties en volume d'eau pour la dissolution d'une partie en poids de la substance active à température ambiante, en combinaison intime avec une substance qui accèlere la dispersion et qui est facilement soluble dans les liquides intestinaux, quelles particules sont combinées en des noyaux pratiquement homogènes en section transversale avec au moins un composant qui accèlere la désagrégation des noyaux dans les liquides intestinaux, les noyaux portant un revêtement pratiquement résistant aux conditions régnant dans l'estomac mais qui est éliminable par érosion dans les conditions régnant dans l'intestin grêle.

2. Une formulation selon la revendication 1, dans laquelle la substance active est une substance qui exige plus de 1000 parties en volume d'eau pour la dissolution d'une partie en poids de la substance active à température ambiante.

3. Une formulation selon la revendication 1 ou 2, dans laquelle la substance active est choisie dans le groupe consistant en l'indométhacin, la spironolactone, l'ibuprofen, le furosemide, la sulfadiazine, la

18

# 0 080 341

sulfamerazine, la progestérone, la réserpine, l'embonate de pyrvinium, la mofebutazone, l'hydrochlorothiazide, la tétracycline, le tolbutamide, l'acetaminophen, la testostérone, l'acide valproïque, l'oestradiol, l'acetazolamide, l'érythromycine, les sels de fer, l'hydralazine, la carbamazepine, la quinidine et les glucosides cardiaques, par exemple le digoxin.

4. Une formulation selon la revendication 1, dans laquelle le composant qui accélère la désagrégation des noyaux dans les liquides intestinaux est choisi dans le groupe consistant en substances sous la forme de particules, les substances étant insolubles dans les liquides gastriques et intestinaux.

5. Une formulation selon la revendication 4, dans lequelle les substances sous la forme de particules sont choisies dans le groupe consistant en particules lamellaires, par exemple du talc, et/ou des particules de forme compacte, par exemple le silicate d'aluminium, l'oxyde de zinc, l'oxyde de magnésium, le bioxyde de titane, la silice colloidale ou le trisilicate de magnésium.

6. Une formulation selon la revendication 1, dans laquelle la substance accélerant la désagrégation comprend des particules d'une substance qui est facilement soluble dans les liquides intestinaux, par exemple le saccharose, le glucose, le mannitol, le sorbitol ou le lactose.

7. Une formulation selon la revendication 5 et 6, dans laquelle les composants accélerant la désagrégation comprennent une combinaison de talc et de saccharose.

8. Une formulation selon la revendication 1, dans laquelle les particules consistant en une combinaison intime de la substance active et de la substance accélerant la dispersion ont une dimension de 1 à 10 µm, plus spécialement de 2 à 5 µm.

9. Une formulation selon la revendication 8, dans laquelle la substance accélerant la dispersion est une substance tensio-active.

10. Une formulation selon la revendication 9, dans laquelle les particules contiennent la substance tensio-active en proportion de 10% en poids au maximum par rapport à la substance active.

11. Une formulation selon la revendication 9, dans laquelle la substance tensio-active est un détergent anionique du non-anionique.

12. Une formulation selon la revendication 11, dans laquelle la substance tensioactive est choisie dans le groupe consistant en les sels de sodium de sulfates d'alcools gras, les sulfosuccinates, les esters partiels d'acides gras et de surbitannes tels que le monooleate de sorbitanne, les esters partiels d'acides gras et de sorbitannes polyhydroxyéthylénés comme le monooléate de polyéthylène-glycol-sorbitanne ou un éther d'alcool gras polyhydroxyéthyléné tel que l'éther laurylique polyhydroxyéthyléné (23).

13. Une formulation selon la revendication 12, dans laquelle la substance tensio-active est le laurylsulfate de sodium.

14. Une formulation selon la revendication 1, dans laquelle le revêtement éliminable par érosion est un revêtement entérique.

15. Une formulation selon la revendication 14, dans laquelle le revêtement entérique est choisi dans le groupe consistant en les polymères et copolymères acryliques, par exemple un polymère de l'acide méthacylique et du méthacrylate de méthyle, la gomme laque, des esters acétiques de la cellulose tels que des esters partiels mixtes de cellulose contenant des groupes phtalate, des groupes acétyle et des groupes acides libres (acétate-phtalate de cellulose), des esters de l'acétate de polyvinyle tels que le phtalate-acétate de polyvinyle, des esters de l'hydroxypropylméthylcellulose comme le phtalate d'hydroxypropylméthylcellulose ou des esters-éthers d'alkylèneglycols de copolymères tels que l'ester partiel de l'éther monométhylique de l'éthylène-glycol d'un copolymère acrylate d'éthyle-anhydride maléique, l'ester de l'éther monométhylique du propylène-glycol et d'un copolymère acrylate d'éthyle-anhydride maléique, l'ester de l'éther monométhylique du dipropylène-glycol et d'un copolymère acrylate d'éthyle-anhydride maléique ou l'ester de l'éther monométhylique du diéthylène-glycol et d'un copolymère acrylate de méthyle-anhydride maléique, un copolymère acrylate de N-butyle-anhydride maléique, un copolymère acrylate d'isobutyle-anhydride maléique ou un copolymère acrylate d'éthyle-anhydride maléique.

16. Une formulation selon la revendication 15, dans laquelle le revêtement est un revêtement qui est éliminé sélectivement par érosion dans la partie distale de l'intestin grêle.

17. Une formulation selon la revendication 16, dans laquelle le revêtement entérique est pratiquement insoluble aux pH inférieurs à 7.

18. Une formulation selon la revendication 17, dans laquelle le revêtement entérique est un revêtement que libérera au moins 90% de la substance active en 1 heure dans 250 ml d'un tampon de phosphate, 0,05M, à pH 7,5, à 37±0,1°C et à une vitesse de rotation de 30±1 r.p.m.

19. Une formulation selon la revendication 17 ou 18, dans laquelle le revêtement entérique comprend un polymère acrylique.

20. Une formulation selon la revendication 19, dans laquelle le revêtement entérique comprend un polymérisate de l'acide méthacrylique et du méthacrylate de méthyle ou de leurs mélanges.

21. Une formulation selon la revendication 1, dans laquelle le diamètre de chacun des moyaux revêtus est de 0,4 à 1,2 mm, plus particuliérement de 0,5 à 1,0 mm, par exemple de 0,5 à 0,7 mm.

22. Une formulation selon la revendication 21, dans laquelle la quantité du revêlement entérique est de 2 à 25% en poids, par rapport au poids total des noyaux.

23. Une formulation selon la revendication 22, dans laquelle les noyaux ont chacun un diamètre de 0,5 à 0,6 mm, et la quantité du revêtement entérique appliquée est de 4 à 12% en poids, par rapport au poids total des noyaux.

24. Un procédé pour la préparation d'une formulation pharmaceutique à unitée multiples à libération contrôlée pour l'administration par voie orale ou d'unitée pour la formulation, dans laquelle une substance active qui exige plus de 30 parties en volume d'eau pour la dissolution d'une partie en poids de la substance active à température ambiante, est broyée avec une substance qui accélère la dispersion et qui est facilement soluble dans les liquides intestinaux, pour obtenir des particules dans lesquelles la substance active est combinée intimement avec la substance accélerant la dispersion, les particules sont combinées en des moyaux pratiquement homogènes en section transverse avec au moins un composant qui accérère la desagrégation des noyaux dans les liquides intestinaux, les noyaux sont revêtus avec un revêtement pratiquement résistant aux conditions régnant dans l'estomac mais qui est éliminable par érosion dans les conditions régnant dans l'intestin grêté, et une multiplicité des noyaux revêtus sont facultativement combinée en une formulation de capsules ou de comprimés.

**Revendications pour l'Etat Contractant: AT**

1. Un procédé pour la préparation d'une formulation pharmaceutique à unités multiples à libération contrôlée pour l'administration par voie orale ou d'unités pour la formulation, dans laquelle une substance active qui exige plus de 30 parties en volume d'eau pour la dissolution d'une partie en poids de la substance active à température ambiante, est broyée avec une substance qui accélère la dispersion et qui est facilement soluble dans les liquides intestinaux, pour obtenir des particules dans lesquelles la substance active est combinée intimement avec la substance accélerant la dispersion, les particules sont combinées en des noyaux pratiquement homogènes en section transversale avec au moins un composant qui accélère la désagrégation des noyaux dans les liquides intestinaux, les noyaux sont revêtus avec un revêtement pratiquement résistant aux conditions régnant dans l'estomac mais qui est éliminable par érosion dans les conditions régnant dans l'intestin grêle, et une multiplicité des noyaux revêtus sont facultativement combinée en une formulation de capsules ou de comprimée.

2. Un procédé selon la revendication 1, dans laquelle la substance active est une substance qui exige plus de 1000 parties en volume d'eau pour la dissolution d'une partie en poids de la substance active à température ambiante.

3. Un procédé selon la revendication 1 ou 2, dans laquelle la substance active est choisie dans le groupe consistant en l'indométhacin, la spironolactone, l'ibuprofen, le furosemide, la sulfadiazine, la sulfamerazine, la progestérone, la réserpine, l'embonate de pyrvinium, la mofebutazone, l'hydrochlorothiazide, la tétracycline, le tolbutamide, l'acetaminophen, la testérone, l'acide valproïque, l'oestradiol, l'acetazolamide, l'érythromycine, les sels de fer, l'hydralazine, la carbamazepine, la quinidine et les glucosides cardiaques, par example le digoxin.

4. Un procédé selon la revendication 1, dans laquelle le composant qui accélère la désagrégation des noyaux dans les liquides intestinaux est choisi dans le groupe consistant en substances sous la forme de particules, les substances étant insolubles dans les liquides gastriques et intestinaux.

5. Un procédé selon la revendication 4, dans laquelle les substances sous la forme de particules sont choisies dans le groupe consistant en particules lamellaires, par exemple du talc, et/ou des particules de forme compacte, par exemple le silicate d'aluminium, l'oxyde de zinc, l'oxyde de magnésium, le bioxyde de titane, la silice colloïdale ou le trisilicate de magnésium.

6. Un procédé selon la revendication 1, dans laquelle la substance accélerant la désagrégation comprend des particules d'une substance qui est facilement soluble dans les liquides intestinaux, par exemple le saccharose, le glucose, le mannitol, le sorbitol ou le lactose.

7. Un procédé selon la revendication 5 et 6, dans laquelle les composants accélerant la désagrégation comprennent une combinaison de talc et de saccharose.

8. Un procédé selon la revendication 1, dans laquelle les particules consistant en une combinaison intime de la substance active et de la substance accélerant la dispersion ont une dimension de 1 à 10 µm, plus spécialement de 2 à 5 µm.

9. Un procédé selon la revendication 8, dans laquelle la substance accélerant la dispersion est une substance tensio-active.

10. Un procédé selon la revendication 9, dans laquelle les particules contiennent la substance tensio-active en proportion de 10% en poids au maximum par rapport à la substance active.

11. Un procédé selon la revendication 9, dans laquelle la substance tensio-active est un détergent anionique ou non-ionique.

12. Un procédé selon la revendication 11, dans laquelle la substance tensio-active est choisie dans le groupe consistant en les sels de sodium de sulfates d'alcools gras, les sulfosuccinates, les esters partiels d'acides gras et de sorbitannes tels que le monooléate de sorbitanne les partiels d'acides gras et de sorbitannes polyhydroxyéthylénés comme le monooléate de polyéthylène-glycol-sorbitanne ou un éther d'alcool gras polyhydroxyéthyléné tel que l'ether laurylique polyhydroxyéthyléné (23).

13. Un procédé selon la revendication 12, dans laquelle la substance tensio-active est le laurylsulfate de sodium.

20

**0 080 341**

14. Un procédé selon la revendication 1, dans laquelle le revêtement eliminable par erosion est un revêtement entérique.

15. Un procédé selon la revendication 14, dans laquelle le revêtement entérique est choisi dans le groupe consistant en les polyméres et copolyméres acryliques, par exemple un polymére de l'acide méthacrylique et du méthacrylate de méthyle, le gomme laque, des esters acétiques de la cellulose tels que des esters partiels mixtes de cellulose contenant des groupes phtalate, des groupes acétyle et des groupes acides libres (acétate-phthalate de cellulose), des esters de l'acétate de polyvinyle tels que le phtalate-acétate de polyvinyle, des esters de l'hydroxypropylméthylcellulose comme le phtalate d'hydroxypropylméthylcellulose ou des esters-éthers d'alkyléne-glycols de copolyméres tels que l'ester partiel de l'éther monométhylique de l'éthylène-glycol d'un copolymére acrylate d'éthyle-anhydride maléique, l'ester de l'éther monométhylique de propylène-glycol et d'un copolymére acrylate d'éthyle-anhydride maléique, l'ester de l'éther monométhylique de dipropylène-glycol et d'un copolymére acrylate d'éthyle-anhydride maléique ou l'ester de l'éther monométhylique de diéthylène-glycol et d'un copolymére acrylate de méthyle-anhydride maléique, un copolymére acrylate de N-butyle-anhydride maléique, un copolymére acrylate d'isobutyle-anhydride maléique ou un copolymère acrylate d'éthyle-anhydride maléique.

16. Un procédé selon la revendication 15, dans laquelle le revêtement est un revêtement qui est éliminé sélectivement par érosion dans la partie distale de l'intestin grêle.

17. Un procédé selon la revendication 16, dans laquelle le revêtement entérique est practiquement insoluble aux pH inférieure à 7.

18. Un procédé selon la revendication 17, dans laquelle le revêtement entérique est on revêtement que libérera au moins 90% de la substance active en l'heure dans 250 ml d'un tampon de phosphate, 0,05M, à pH 7,5, à 37±0,1°C et une vitesse de rotation de 30±1 r.p.m.

19. Un procédé selon la revendication 17 ou 18, dans laquelle le revêtement entérique comprend un polymére acrylique.

20. Un procédé selon la revendication 19, dans laquelle le revêtement entérique comprend on polymérisate de l'acide méthacrylique et du méthacrylate de méthyle ou de leurs mélanges.

21. Un procédé selon la revendication 1, dans laquelle le diamètre de chacun des noyaux revêtus est de 0,4 à 1,2 mm, plus particulièrement de 0,5 à 1,0 mm, par exemple de 0,5 à 0,7 mm.

22. Un procédé selon la revendication 21, dans laquelle la quantité du revêtement entérique est de 2 à 25% en poids, par rapport au poids total des noyaux.

23. Un procédé selon la revendication 22, dans laquelle les noyaux ont chacun un diamètre de 0,5 à 0,6 mm, et lu quantité du revêtement entérique appliquée est de 4 à 12% en poids, par rapport au poids total des noyaux.

# Fig. 1.

Fig. 2.